# EUROPEAN PATENT SPECIFICATION

(11) **EP 3 220 939 B1**
(45) Date of publication and mention of the grant of the patent: **30.06.2021**
(21) Application number: 15795157.5
(22) Date of filing: 16.11.2015
(51) Int. Cl.: A61K 38/08, A61P 3/10, A61P 3/08

(54) **HOMODIMERIC PEPTIDE FOR TREATING DIABETES AND RELATED DISEASES**
HOMODIMER-PEPTID ZUR BEHANDLUNG VON DIABETES UND VERWANDTEN ERKRANKUNGEN
PEPTIDE HOMODIMÉRIQUE POUR LE TRAITEMENT DU DIABÈTE ET DES MALADIES LIÉES

(30) Priority: 18.11.2014 GB 201420445
(43) Date of publication of application: 27.09.2017
(73) Proprietor: Rogers, Arpi, London NW3 7AJ (GB)
(72) Inventor: Rogers, Arpi, London NW3 7AJ (GB)
(74) Representative: Chapman, Desmond Mark
(86) International application number: PCT/EP2015/076712
(87) International publication number: WO 2016/079066

(56) References cited:
- WO-A2-2007/017686
- None

### SEQUENCE LISTING

<110> ROGERS, Arpi
<120> Therapeutic homodimer and uses thereof
<141> 2015-11-16
<160> 5
<170> SeqWin2010, version 1.0
<210> 1
   <211> 10
   <212> PRT
   <213> Artificial Sequence
<220>
   <223> peptide
<400> 1
   Cys Gln Gln Tyr Asn Ser Tyr Pro Leu Thr
   1               5                   10
<210> 2
   <211> 17
   <212> PRT
   <213> Artificial Sequence
<220>
   <223> peptide
<400> 2
   Asn Ile Tyr Pro Ser Asp Ser Tyr Thr Asn Tyr Asn Gln Lys Phe Lys
   1               5                   10                  15
   Asp
<210> 3
   <211> 18
   <212> PRT
   <213> Artificial Sequence
<220>
   <223> peptide
<400> 3
   Cys Asn Ile Tyr Pro Ser Asp Ser Tyr Thr Asn Tyr Asn Gln Lys Phe
   1               5                   10                  15
   Lys Asp
<210> 4
   <211> 8
   <212> PRT
   <213> Artificial Sequence
<220>
   <223> peptide
<400> 4
   Leu Arg Gly Leu Leu Pro Asp Tyr
   1               5
<210> 5
   <211> 9
   <212> PRT
   <213> Artificial Sequence
<220>
   <223> peptide
<400> 5
   Cys Leu Arg Gly Leu Leu Pro Asp Tyr
   1               5

## Description

### FIELD OF THE INVENTION

The invention relates to therapeutic peptides and uses thereof. In particular, the invention relates to the use of a homodimer of disulphide-linked monomers, or a pharmaceutically acceptable salt thereof, in the treatment or prevention of diabetes and related diseases and conditions in mammalian subjects, via oral administration of the homodimer.

### BACKGROUND

Bioavailability and stability are key factors in drug delivery, and influence the choice of drug and drug delivery route. Oral administration is normally the most preferred route, for example due to patient convenience and compliance, but this route is especially challenging for therapeutic peptides. Peptide drugs generally have poor permeability through mucosal membranes, particularly the oral and intestinal mucosa. Furthermore, peptides normally suffer from poor stability under physiological conditions. In particular, peptides are usually subject to degradation by peptidases and proteases of the gut and plasma. Successful oral (peroral or intraoral) delivery of therapeutic peptides generally requires specialised peptides and/or bespoke delivery formulations, where the peptide itself is chemically modified to improve its bioavailability or stability, or where the excipients are designed, adapted or selected to improve the peptide's bioavailability or stability under physiological conditions. For example, efforts to deliver therapeutic peptides orally have involved the use of various functional excipients such as enzyme inhibitors (*e.g*. protease or peptidase inhibitors, such as bacitracin, nafamostat mesilate, camostat mesilate, sodium glycholate, Aprotinin, Bestatin, Pepstatin, PMSF, Leupeptin, and the like), absorption enhancers, bioadhesive polymers, particularly mucoadhesive polymers (*e.g*. chitosan), and transporter molecules. Despite the interest in oral delivery of therapeutic peptides, and despite continuing efforts to identify ways to improve the oral bioavailability and stability of peptides, the great majority of therapeutic peptides are still administered parenterally, *e.g.* by injection. Potential problems for peptide therapeutics are highlighted in several textbooks (*e.g*. "Therapeutic Peptides and Protein Formulation: Processing and Delivery Systems", A. K. Banga, 2005; Delivery Technologies for Biopharmaceuticals: Peptides, Proteins, Nucleic Acids and Vaccines, Jorgensen & Nielsen, 2009; Mucosal Delivery of Biopharmaceuticals: Biology, Challenges and Strategies, das Neves & Sarmento, 2014). Transmucosal delivery, and in particular oral delivery, of therapeutic peptides remains a major challenge.

Diabetes is a disease in which a hyperglycaemic state persists due to an acute or chronic decrease in the action of insulin, resulting in disorders of sugar metabolism, lipid metabolism, and amino acid metabolism. Diabetes is associated with complications in the eyes, kidneys, nervous system, cardiovascular system, skin, and other areas. Although certain therapies exist for diabetes and related diseases and conditions, these diseases and conditions remain problematic and are a significant cause of morbidity and mortality.

A number of peptide therapies for diabetes have already received regulatory approval for human clinical use (*e.g*. Liraglutide and Lixisenatide are GLP-1 R agonists approved for the treatment of Type 2 diabetes), and additional peptide drugs are under development (*e.g*. as reviewed in Vlieghe et a/., Drug Discovery Today, January 2010, Vol. 15, pages 40-56 and Kaspar & Reichert, Drug Discovery Today, July 2013, Vol. 18, pages 807-817; see also commonly-owned patent application WO 2007/017686). WO 2007/017686 describes a mixture of three peptides, including the F90 homodimer (CQQYNSYPLT), for use in a method of treating glucose intolerance and type 2 diabetes through intra muscular administration (e.g. see Examples 6 and 7). However, there remains a need for novel therapies for diabetes and related diseases and conditions.

### SUMMARY OF THE INVENTION

The invention relates to the therapeutic use of a peptide drug. In particular, the invention relates to the therapeutic use of a homodimer consisting of covalently linked monomer peptides, each monomer consisting of the amino acid sequence CQQYNSYPLT (SEQ ID NO:1), wherein the monomer peptides are linked to form the homodimer by a disulphide bond between the N-terminal cysteine residues of the monomers. It is postulated by the inventor, without wishing to be bound by the theory, that the homodimer used in the invention benefits from functional properties which render it surprisingly suitable for transmucosal administration, and in particular that it benefits from unexpected transmucosal bioavailability and/or unexpected stability under physiological conditions following transmucosal administration.

In a first aspect, the invention provides a homodimer consisting of covalently linked monomer peptides, each monomer consisting of the amino acid sequence CQQYNSYPLT (SEQ ID NO:1), wherein the monomer peptides are linked by a disulphide bond between the N-terminal cysteine residues of the monomers, or a pharmaceutically acceptable salt thereof, for use in the treatment or prevention of hyperinsulinaemia, hyperglucagonaemia, glucose intolerance, and/or insulin resistance in a mammalian subject by oral administration.

In a second aspect, the invention provides a pharmaceutical composition comprising: (i) a homodimer consisting of covalently linked monomer peptides, each monomer consisting of the amino acid sequence CQQYNSYPLT (SEQ ID NO:1), wherein the monomer peptides are linked by a disulphide bond between the N-terminal cysteine residues of the monomers, or a pharmaceutically acceptable salt thereof; and (ii) one or more pharmaceutically acceptable excipients, for use in the treatment or prevention of hyperinsulinaemia, hyperglucagonaemia, glucose intolerance, and/or insulin resistance in a mammalian subject by oral administration. The pharmaceutically acceptable excipients used in the invention do not provide pharmacological activity or other direct effect in the treatment or prevention of the targeted disease or condition, but may act to improve the homodimer's pharmacological activity when the homodimer is administered to a mammalian subject in conjunction with the excipient. The invention envisages the use of functional and/or non-functional excipients, as explained elsewhere herein.

Other features and advantages of the present invention will become apparent from the following detailed description. It should be understood, however, that the detailed description and the specific examples, while indicating particular features and embodiments of the invention, are given by way of illustration only.

### DETAILED DESCRIPTION OF THE INVENTION

The invention relates to the therapeutic use of a peptide drug. In particular, the invention relates to the therapeutic use of a homodimer consisting of covalently linked monomer peptides.

### The homodimer

The invention relates to a homodimer consisting of covalently linked monomer peptides, each monomer consisting of the amino acid sequence CQQYNSYPLT (SEQ ID NO:1), wherein the monomer peptides are linked to form the homodimer by a disulphide bond between the N-terminal cysteine residues of the monomers.

The homodimer used in the present invention was first described in commonly owned patent application WO 2007/017686. Although the homodimer is not a naturally-occurring molecule, it contains only naturally-occurring amino acids and linkages. For example, all of the amino acids of the homodimer are standard amino acids in the L form. Furthermore, the amino acid residues of the monomer peptides are linked exclusively by peptide bonds, and the homodimer does not comprise any C-terminal modifications. As used herein, references to "the homodimer" are references to the unmodified homodimer, *i.e.* a homodimer consisting of covalently linked monomer peptides, each monomer consisting of the amino acid sequence CQQYNSYPLT (SEQ ID NO:1), wherein: (i) all of the amino acids of the homodimer are standard amino acids in the L form, (ii) the amino acid residues of the monomer peptides are linked exclusively by peptide bonds, (iii) the monomer peptides are linked to form the homodimer by a disulphide bond between the N-terminal cysteine residues of the monomers, and (iv) and the homodimer does not comprise any C-terminal modifications.

In some embodiments of the invention the homodimer is administered to the mammalian subject.

However, as explained elsewhere herein, in other embodiments of the present invention a pharmaceutically acceptable salt of the homodimer is administered to the mammalian subject.

It is postulated by the inventor, without wishing to be bound by the theory, that the homodimer used in the invention benefits from functional properties which render it surprisingly suitable for transmucosal administration, and in particular that it benefits from unexpected transmucosal bioavailability and/or unexpected stability under physiological conditions following transmucosal administration.

### Preparation of the monomer peptides

The monomer peptides used in the production of the homodimer (or a pharmaceutically acceptable salt thereof) may be produced using any suitable method. Such methods include chemical synthesis and recombinant expression, and also enzymatic or chemical cleavage of polypeptides containing one or more copies of the monomer sequences.

### Chemical synthesis

The monomer peptides used in the invention may be produced by chemical synthesis, using any suitable chemical synthesis method. Methods for synthetic production of peptides are well known in the art. Detailed descriptions as well as practical advice for producing synthetic peptides may be found in various textbooks (*e.g*. Synthetic Peptides: A User's Guide (Advances in Molecular Biology), Grant G. A., Oxford University Press, 2002, or Pharmaceutical Formulation: Development of Peptides and Proteins, Frokjaer and Hovgaard, Taylor and Francis, 1999).

The monomer peptides may be produced using various solid-phase synthesis techniques known to the skilled person. For example, t-Boc or FMOC-based chemistries may be used in solid phase peptide synthesis methods (*e.g*. see "Solid Phase Peptide Synthesis", eds. Stewart & Young, available from Pierce Chem. Co). Alternatively, solution phase synthesis may be applied (*e.g*. see "Chemical Approaches to the Synthesis of Peptides and Proteins", Lloyd-Williams, P., Albericio, F. and Giralt, E., CRC Press, 1997).

### Recombinant or cell-free expression

The monomer peptides used in the invention will normally be produced by chemical synthesis, rather than by expression from a nucleic acid, but it is also possible to produce the monomer peptides via cellular or cell-free recombinant expression systems. The skilled person is aware of numerous expression systems available for expression of a nucleic acid encoding peptides and proteins. Appropriate recombinant nucleic acids (*e.g*. expression vectors containing coding and regulatory sequences) can be obtained using any number of methodologies known to those of skill in the art (e.g. as described in the latest edition of Sambrook - Molecular Cloning: A Laboratory Manual). Host cells used in the production of the monomer peptides may be transformed, transfected or transduced with appropriate expression vectors, and the host cells may be prokaryotic or eukaryotic. Cells useful for production of the monomer peptides (*e.g*. bacterial cells, yeast cells, mammalian cells, insect cells, plant cells) are known and/or available, for instance, from the American Type Culture Collection Catalogue of Cell Lines and Hybridomas (www.atcc.org), or other known or commercial sources. The medium used to culture the host cells may be any conventional medium. Suitable culture media are also available from commercial suppliers or may be prepared according to published recipes.

### Purification and analysis

The monomer peptides may be purified, to the desired degree of purity, by any suitable method, such as preparative HPLC, after they are produced. For example, peptides recombinantly produced in cells may be recovered from the culture medium by conventional procedures including separating the host cells from the medium by centrifugation or filtration, precipitating the peptides components of the supernatant or filtrate by means of a salt, *e.g*. ammonium sulphate, purification by a variety of chromatographic procedures, *e.g*. HPLC, ion exchange chromatography, affinity chromatography, *etc.*

The purified monomer peptides may be analysed using any appropriate method, after purification. For example, the monomer peptides may be analysed by HPLC or mass spectrometry.

### Preparation of the homodimer

Once produced, and purified to the desired degree of purity, the monomer peptides may be used to produce the homodimer (or a pharmaceutically acceptable salt thereof) by forming a disulphide bond between the N-terminal cysteine residues of the monomer peptides. The disulphide bond may be formed by oxidation of the sulfhydryl (-SH) groups of the N-terminal cysteine residues, e.g. by taking advantage of the natural tendency of cysteine residues to dimerise in acidic aqueous solutions to form cystine residues. Suitable methods for disulphide linking of the monomer peptide are known in the art (*e.g*. Methods in Molecular Biology, Vol. 35, Peptide Synthesis Protocols, Chapter 7: Formation of Disulfide Bonds in Synthetic Peptides and Proteins, Andreu et al., 1994).

### Purification and analysis

The homodimer (or a pharmaceutically acceptable salt thereof) may be purified, to the desired degree of purity, by any suitable method (*e.g*. preparative HPLC), and the purified homodimer or pharmaceutically acceptable salt may be analysed using any appropriate method. For example, the purified homodimer may be analysed by HPLC or mass spectrometry.

### Good Manufacturing Practice (GMP)

The monomer peptides and homodimer (or pharmaceutically acceptable salt) may be produced according to Good Manufacturing Process (GMP) rules, *i.e.* in full compliance with the GMP regulations of the United States Food and Drug Administration (FDA).

### Pharmaceutically acceptable salts

As noted elsewhere herein, in some embodiments of the invention the homodimer is administered to the mammalian subject. However, in other embodiments a pharmaceutically acceptable salt of the homodimer is administered to the mammalian subject.

Pharmaceutically acceptable salts include acid addition salts (formed with the free amino groups of peptide compounds) and which are formed with inorganic acids such as, for ex-ample, hydrochloric or phosphoric acids, or such organic acids as acetic acid, oxalic acid, tartaric acid, mandelic acid, and the like. Salts formed with the free carboxyl group may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

Pharmaceutically acceptable salts may be prepared by well-known methods. The salts may be formed by conventional means, such as by reacting a free base form with one or more equivalents of the appropriate acid in a solvent or medium in which the salt is insoluble, or in a solvent such as water which is removed in vacuo or by freeze drying or by exchanging the anions of an existing salt for another anion on an ion exchange resin. For a review of pharmaceutically acceptable salts, see Stahl and Wermuth, Handbook of Pharmaceutical Salts: Properties, Selection and Use (Wiley-VCH, Weinheim, Germany, 2002).

As noted elsewhere herein, susceptibility to cleavage by enzymes such as proteases or peptidases is normally a major problem for peptide drugs, especially for oral administration. When susceptibility of a peptide drug to cleavage by proteases or peptidases is a problem, replacement of sensitive peptides bonds with a non-cleavable alternative, or the replacement of an L-amino acid residue with a D-amino acid residue, or the use of modified N-terminal or C-terminal groups, is sometimes used to stabilise the peptide for therapeutic use. These strategies are known in the art.

It has surprisingly been found that the homodimer used in the present invention is therapeutically effective in mammalian subjects without chemical modifications intended to protect the homodimer from degradation by enzymes, such as peptidases or proteases. Although the homodimer is not a naturally-occurring molecule, it contains only naturally-occurring amino acids (all of the amino acids of the homodimer are standard amino acids in the L form). Furthermore, the amino acid residues of the monomer peptides are linked exclusively by peptide bonds, and the homodimer does not comprise any C-terminal modifications.

### Pharmaceutical compositions

Once produced and purified, as appropriate for the desired degree of purity, the homodimer will normally be formulated into a pharmaceutical composition by combining it with one or more pharmaceutically acceptable excipients. However, the homodimer may in some embodiments be administered alone without a pharmaceutically acceptable excipient, *i.e.* packaged as a drug product in the absence of any pharmaceutically acceptable excipients. In some embodiments, the homodimer is the sole ingredient administered to the subject, *i.e.* the homodimer is packaged as a drug product in the absence of any other ingredients. When an excipient is used, the invention envisages the use of pharmaceutical compositions comprising functional excipients and/or non-functional excipients, as explained elsewhere herein.

The invention involves the use of the homodimer as an active ingredient. The term "active ingredient", as used herein, refers to any component of a drug product that provides a pharmacological activity or other direct effect in the treatment or prevention of a targeted disease or condition. In some embodiments, the homodimer is used as the sole ingredient of the drug product administered to the mammalian subject. In some embodiments, the homodimer is used as the sole active ingredient in the drug product administered to the mammalian subject. In other embodiments, the drug product administered to the mammalian subject comprises the homodimer in conjunction with one or more other active ingredients. In some embodiments, the drug product containing the homodimer is co-administered to the mammalian subject with a separate drug product containing one or more other active ingredients for combination therapy, as explained elsewhere herein.

The term "pharmaceutical composition" as used herein, refers to a formulation containing the homodimer as an active ingredient, in association with one or more pharmaceutically acceptable excipients, and optionally in association with one or more other active ingredients, and is not limited to the finished dosage form (drug product) as administered to the patient.

The term "drug product", as used herein, refers to the finished dosage form that contains the homodimer as an active ingredient, optionally in association with one or more pharmaceutically acceptable excipients and/or optionally in association with one or more other active ingredients, as administered to the patient.

### Excipients

The term "excipient", as used herein, refers to any components of a drug product other than the active ingredient(s). The term "excipient" therefore refers to any components of a drug product which do not provide pharmacological activity or other direct effect on a targeted disease or condition.

A "pharmaceutically acceptable" excipient is an excipient which is compatible with the other components of the composition and which is not deleterious to the mammalian subject (*e.g*. it is nontoxic). The term "pharmaceutically acceptable excipient" includes excipients suitable for veterinary use, as well as excipients suitable for human use.

Suitable excipients for use in humans are described in the most recent editions of Remington: The Science and Practice of Pharmacy and Aulton's Pharmaceutics: The Design and Manufacture of Medicines, which are both standard reference texts. Examples of types of excipients include fillers, extenders, diluents, wetting agents, solvents, emulsifiers, preservatives, flavours, absorption enhancers, bioadhesives such as mucoadhesives, enzyme inhibitors, sustained-release matrices, and colouring agents, and others.

### "Functional" and "non-functional" excipients

The invention envisages the use of pharmaceutical compositions comprising functional excipients and/or non-functional excipients.

As used herein, the term "functional excipient" refers to an excipient which acts to improve the homodimer's pharmacological activity when the homodimer is administered to a mammalian subject in conjunction with the excipient. A "functional excipient" may additionally improve the manufacture, transport, storage and/or administration of the drug product. The term "functional excipient" refers not only to any excipient which has been specifically designed, adapted or selected to improve the pharmacological activity of the homodimer, nor only to any excipient which is recognised in the art as improving the pharmacological activity of therapeutic peptides, but to any excipient which inherently improves the pharmacological activity of the homodimer when the homodimer is administered to a mammalian subject in conjunction with the excipient.

A "non-functional excipient" is any excipient which is not a functional excipient as defined herein. Therefore, as used herein, a "non-functional excipient" is any excipient which does not act to improve the homodimer's pharmacological activity when the homodimer is administered to a mammalian subject in conjunction with the excipient, but a "non-functional excipient" may improve the manufacture, transport, storage and/or administration of the homodimer.

In some embodiments of the invention, the homodimer is formulated or administered in a pharmaceutical composition in the absence of any "functional" excipients. In these embodiments, the homodimer is optionally formulated or administered in a composition that contains one or more "non-functional" excipients. The homodimer may be formulated or administered in a pharmaceutical composition that only comprises "non-functional" excipients in addition to the active ingredient(s). In these embodiments, small amounts of functional excipients may be present in the compositions administered to the mammalian subject, provided that they are not present in an amount sufficient to improve the homodimer's pharmacological activity.

In other embodiments of the invention, the homodimer is formulated or administered in the presence of one or more "functional" excipients, in amounts sufficient to improve the homodimer's pharmacological activity.

In other embodiments, the compositions used in the invention contain one or more functional excipients and one or more non-functional excipients.

It has surprisingly been found by the inventor that the homodimer is therapeutically effective in oral administration to mammalian (in particular, human) subjects when formulated without any functional excipients that improve the transmucosal bioavailability of the homodimer when the homodimer is administered to a mammalian subject in conjunction with the excipient. Therefore, in some embodiments of the invention, the homodimer is formulated or administered in the absence of any functional excipients which improve the transmucosal bioavailability of the homodimer when the homodimer is administered to a mammalian subject in conjunction with the excipient. In these embodiments, the homodimer may be formulated or administered in the absence of any excipient which improves the oral bioavailability of the homodimer. The homodimer may be formulated or administered in the absence of any excipient which improves the peroral bioavailability of the homodimer. The homodimer may be formulated or administered in the absence of any excipient which improves the intraoral bioavailability of the homodimer. In these embodiments, the excipients which are absent from the composition are likewise not only any excipient which has been specifically designed, adapted or selected to improve the transmucosal bioavailability of the homodimer, nor only any excipient which is recognised in the art as improving the transmucosal bioavailability of the homodimer, but also any excipient which inherently improves the transmucosal bioavailability of the homodimer when the homodimer is administered to a mammalian subject in conjunction with the excipient. Similarly, small amounts of such functional excipients may be present in the compositions administered to the mammalian subject, provided that they are not present in an amount sufficient to improve the transmucosal bioavailability of the homodimer.

Types of functional excipients commonly used in the art to improve the transmucosal bioavailability of peptide drugs include, but are not limited to, absorption enhancers (also known as permeation enhancers or penetration enhancers; e.g. bile salts and acids, fatty acids, surfactants, acylcarnitines, phospholipids, cyclodextrins, cationic or anionic polymers, thiolated polymers, synthetic peptides, and the like), bioadhesives, such as mucoadhesives (*e.g*. chitosan and its derivatives, such as trimethylchitosan and thiolated chitosan, pectin, alginate and its derivatives, such as sodium alginate, cellulose derivatives, such as hydroxypropyl cellulose, hydroxypropylmethyl cellulose (HPMC), hydroxyethylcellulose, methylcellulose (MC) and sodium carboxymethyl cellulose (CMC-Na), hyaluronic acid, polycarbophil, carbopol/carbomer, gums, such as xanthan gum, guar gum, hydroxypropyl guar gum and carrageenan, and various copolymers (e.g. poly(hydroxytheyl methacrylate) and the like), particulate carriers (*e.g*. polymer or lipid microparticles, polymer or lipid nanoparticles, lipid microemulsions, liposomes, and the like) and covalently or non-covalently linked transporter molecules (*e.g*. cell-penetrating peptides such as L-penetrin and the like).

In some embodiments of the invention, one or more of these recited types of functional excipients is specifically excluded from use in the pharmaceutical compositions. For example, in some embodiments, the homodimer may be formulated or administered in the absence of any absorption enhancers, and in particular any mucosal absorption enhancers. The homodimer may also be formulated or administered in the absence of any bioadhesives, such as in the absence of any mucoadhesives. The homodimer may also be formulated or administered in the absence of any covalently or non-covalently linked transporter molecules. The homodimer may also be formulated or administered in the absence of any absorption enhancers, bioadhesives, and covalently or non-covalently linked transporter molecules. In some embodiments, the homodimer is formulated or administered in the absence of any excipient selected from absorption enhancers (also known as permeation enhancers or penetration enhancers; *e.g*. bile salts and acids, fatty acids, surfactants, acylcarnitines, phospholipids, cyclodextrins, cationic or anionic polymers, thiolated polymers, synthetic peptides, and the like), bioadhesives, such as mucoadhesives (*e.g*. chitosan and its derivatives, such as trimethylchitosan and thiolated chitosan, pectin, alginate and its derivatives, such as sodium alginate, cellulose derivatives, such as hydroxypropyl cellulose, hydroxypropylmethyl cellulose (HPMC), hydroxyethylcellulose, methylcellulose (MC) and sodium carboxymethyl cellulose (CMC-Na), hyaluronic acid, polycarbophil, carbopol/carbomer, gums, such as xanthan gum, guar gum, hydroxypropyl guar gum and carrageenan, and various copolymers (e.g. poly(hydroxytheyl methacrylate) and the like), particulate carriers (*e.g*. polymer or lipid microparticles, polymer or lipid nanoparticles, lipid microemulsions, liposomes, and the like) and covalently or non-covalently linked transporter molecules (*e.g*. cell-penetrating peptides such as L-penetrin and the like).

However, it is envisaged that the therapeutic efficacy of the homodimer may be improved by the use of a functional excipient which improves the transmucosal bioavailability of the homodimer when the homodimer is administered to a mammalian subject in conjunction with the excipient. Therefore, in other embodiments, the homodimer is formulated or administered in the presence of one or more functional excipients which improve the transmucosal bioavailability of the homodimer when the homodimer is administered to a mammalian subject in conjunction with the excipient. In these embodiments, the homodimer may be formulated or administered in the presence of one or more excipients which improve the oral bioavailability of the homodimer. The homodimer may be formulated or administered in the presence of one or more excipients which improve the peroral bioavailability of the homodimer. The homodimer may be formulated or administered in the presence of one or more excipients which improve the intraoral bioavailability of the homodimer. Types of functional excipients commonly used to improve the transmucosal (*e.g*. oral, peroral or intraoral) bioavailability of peptide drugs include those recited above.

In some embodiments, the homodimer is formulated or administered in the absence of any functional excipient which improves the biostability of the homodimer when the homodimer is administered in conjunction with the excipient (*e.g*. in the absence of any functional excipient which protects the homodimer from enzymatic degradation, for example by peptidases or proteases). In some embodiments, the homodimer is formulated or administered in the absence of an enzyme inhibitor, such as a peptidase or protease inhibitor (*e.g*. in the absence of bacitracin, nafamostat mesilate, camostat mesilate and/or sodium glycholate, Aprotinin, Bestatin, Pepstatin, PMSF, Leupeptin, and the like).

However, it is envisaged that the therapeutic efficacy of the homodimer may be improved by the use of a functional excipient which improves the biostability of the homodimer when the homodimer is administered in conjunction with the excipient (*e.g*. in the presence of any functional excipient which protects the homodimer from enzymatic degradation, for example by peptidases or proteases). Therefore, in other embodiments, the homodimer may be formulated or administered in a composition containing one or more enzyme inhibitors, such as a peptidase or protease inhibitor (*e.g.* in the presence of bacitracin, nafamostat mesilate, camostat mesilate and/or sodium glycholate, Aprotinin, Bestatin, Pepstatin, PMSF, Leupeptin, and the like).

The homodimer may be formulated or administered in the absence of any functional excipients which have adjuvant properties. For example, the homodimer may be formulated or administered in the absence of any mineral salts (*e.g*. aluminium salts), oil emulsions, saponins, virosomes or virus-like particles, bacterial and microbial derivatives (*e.g*. bacterial toxoids), polymer microparticles, liposomes, muramyl peptides, or imidazoquinolone compounds.

However, it is envisaged that the therapeutic efficacy of the homodimer may be improved by the use of an adjuvant. Therefore, in other embodiments, the homodimer may be formulated or administered in a composition containing one or more adjuvants, such as one or more of the types of adjuvants recited above.

In some embodiments, the homodimer is formulated or administered in the absence of any functional excipient which improves the biostability of the homodimer, and in the absence of any excipient which improves the transmucosal (oral, intraoral or peroral) bioavailability of the homodimer (and optionally further in the absence of any functional excipients which have adjuvant properties).

In other embodiments, the homodimer may be formulated or administered in the presence of a functional excipient which improves the biostability of the homodimer, and in the presence of an excipient which improves the transmucosal (oral, intraoral or peroral) bioavailability of the homodimer (and optionally further in the presence of one or more functional excipients which have adjuvant properties).

The pharmaceutical compositions of use in the invention are generated by bringing an appropriate amount of the homodimer into association with appropriate amounts of one or more pharmaceutically acceptable excipients, *e.g*. by mixing. Thus, there is disclosed herein a method for producing a pharmaceutical composition, comprising bringing an appropriate amount of a homodimer into association with appropriate amounts of one or more pharmaceutically acceptable excipients (*e.g*. by mixing), the homodimer consisting of covalently linked monomer peptides, each monomer consisting of the amino acid sequence CQQYNSYPLT (SEQ ID NO:1), wherein the monomer peptides are linked by a disulphide bond between the N-terminal cysteine residues of the monomers. In these methods, the method optionally comprises or does not comprise bringing the homodimer into association with any of the types of excipients or specific excipients described above, as appropriate to produce the relevant pharmaceutical composition.

As noted elsewhere herein, the homodimer may in some embodiments be administered alone without a pharmaceutically acceptable excipient, *i.e.* packaged as a drug product in the absence of any excipient. Thus, there is disclosed herein a method for producing a drug product, comprising packaging an appropriate amount of the homodimer as a drug product in the absence of any pharmaceutically acceptable excipients. In some embodiments, the homodimer is the sole ingredient administered to the subject, *i.e.* the homodimer is packaged as a drug product in the absence of any other ingredients. Thus, also disclosed herein is a method for producing a drug product, comprising packaging an appropriate amount of the homodimer as a drug product in the absence of any other ingredients.

### Sterility

The drug products used in the invention may be sterile, but the use of a sterile drug product may not always be essential depending on the route of administration, and the use of a non-sterile drug product may be advantageous because of the reduced burden during manufacture, transport, storage and administration. Rigorous sterility checking is not always required, and the skilled person will be aware of the circumstances in which rigorous sterility checking is appropriate or required. The skilled person will also be aware of appropriate sterilization methods. In some embodiments, the drug products used in the invention are non-sterile. In other embodiments, the drug products used in the invention are sterile. By "sterile" herein is meant the absence of viable micro-organisms, as determined using routine analysis methods. By "non-sterile" is meant any drug product which is not sterile.

### Routes of Administration

After production of the drug product the homodimer is administered to a mammalian subject. The invention relates to the transmucosal administration of the homodimer, and in particular to the administration of the homodimer by absorption through the oral or gastrointestinal mucosa.

As used herein, the term "transmucosal administration" refers to administration across one or more mucosal surface, and encompasses absorption of the homodimer through the oral (*e.g*. buccal and sublingual), gastrointestinal, nasal, pulmonary, vaginal and rectal mucosa.

In the claimed invention, the homodimer is administered orally, for absorption through the oral mucosa or gastrointestinal mucosa. In some embodiments, the administration of the peptide is by peroral administration. In alternative embodiments, the administration of the peptide is by intraoral administration. Buccal and sublingual delivery are both preferred routes for intraoral administration.

As used herein, the term "oral administration" (or "administered orally") refers to administration to or by way of the mouth, and encompasses both "peroral" administration and "intraoral" administration. The term "peroral" is used herein to refer to an administration in which the homodimer is swallowed and passes through the mouth into the gastrointestinal tract, to be absorbed primarily through the mucosa of the gastrointestinal (GI) tract. The term "intraoral" is used herein to refer to absorption of the homodimer from the mouth itself, through the oral mucosa (e.g. via the buccal, lingual, sublingual and sublabial routes). As used herein, the term "buccal" refers to administration directed toward the cheek, from within the mouth, through the mucosal membranes lining the cheeks (*i.e.* through the buccal mucosa). As used herein, the term "sublingual" refers to administration beneath the tongue, through the mucosal membranes lining the floor of the mouth under the tongue (i.e. through the sublingual mucosa).

### Dosages, Dosage Forms and Dosage Regimens

### Dosages

The invention requires the administration of a therapeutically effective amount or a prophylactically effective amount of the homodimer to a mammalian subject. The term "therapeutically effective amount" as used herein refers to an amount of the homodimer sufficient to treat the targeted disease or condition. The term "prophylactically effective amount" as used herein refers to an amount of the homodimer sufficient to prevent the targeted disease or condition. The term "mammalian subject" takes its normal meaning and includes mice, rats, guinea pigs, hamsters, rabbits, dogs, cats, sheep, goats, horses, pigs, humans and non-human primates such as monkeys and chimpanzees. Both veterinary clinical use and human clinical use of the homodimer and compositions described herein are envisaged. In preferred embodiments, the mammalian subject is a human subject. The term "human subject" refers to any person in need of therapy who may benefit from administration of the homodimer. Use of the homodimer in the treatment or prevention of a disease or condition in birds is also envisaged.

The precise effective amount for a human subject may depend upon the severity of the disease state, general health of the subject, age, weight, and gender of the subject, diet, time and frequency of administration, drug combination(s), reaction sensitivities, and tolerance/response to therapy. Methods for determining useful doses for use in humans are known in the art and preferred amounts can be determined by the clinician via routine experimentation. Therapeutically effective doses may be estimated initially either in cell culture assays or in small animal models.

Generally, an effective dose for a human subject will be from 0.0001 mg/kg to 50 mg/kg, such as from 0.001 mg/kg to 10 mg/kg, from 0.005 mg/kg to 5 mg/kg, or from 0.0075 mg/kg to 2.5 mg/kg, *e.g.* at 0.001 mg/kg or 0.005 mg/kg to 2 mg/kg, to 1.5 mg/kg, to 1.0 mg/kg, to 0.5 mg/kg, or to 0.25 mg/kg. In some embodiments, the homodimer may be administered at a dose of from 0.001 mg/kg to 2 mg/kg, at a dose of from 0.005 mg/kg to 2 mg/kg, or at a dose of from 0.005 mg/kg to 1 mg/kg. Compositions comprising the homodimer at doses of approximately 0.03 mg/kg have been shown to provide useful therapeutic effects in human patients (see the Examples herein), without significant undesirable side-effects. It is envisaged by the inventor that smaller doses, equivalent doses, or larger doses, of the homodimer can be used in the invention. Thus, preferred doses may comprise at least 0.001 mg/kg, at least 0.002 mg/kg, at least 0.003 mg/kg, at least 0.004 mg/kg, at least 0.005 mg/kg, at least 0.006 mg/kg, at least 0.007 mg/kg, at least 0.008 mg/kg, at least 0.009 mg/kg, at least 0.01 mg/kg, at least 0.015 mg/kg, at least 0.02 mg/kg, at least 0.03 mg/kg, at least 0.04 mg/kg, or at least 0.05 mg/kg of the homodimer. Preferred doses may also comprise less than 1 mg/kg, less than 0.9 mg/kg, less than 0.08 mg/kg, less than 0.07 mg/kg, less than 0.06 mg/kg, or less than 0.05 mg/kg of the homodimer.

Effective doses for a human subject may be from 0.007 mg to 3500 mg, such as from 0.07 mg to 700 mg, from 0.35 mg to 350 mg, or from 0.525 mg to 175 mg, *e.g.* at 0.1 mg or 0.35 mg to 140 mg, 105 mg, 70 mg, 35 mg, 17.5 mg, 15 mg, 12.5 mg, 10 mg, 7.5 mg or 5 mg. In some embodiments, the homodimer is used at from 0.1 mg to 10 mg per dose, at from 0.35 mg to 10 mg per dose, or at from 0.35 mg to 5 mg per dose. Compositions comprising the homodimer at doses of approximately 2 mg have been shown to provide useful therapeutic effects in human patients (see the Examples herein), without significant undesirable side-effects.

### Dosage Forms

A "dosage form" is the physical form in which a drug is produced and dispensed as a drug product, such as a tablet, a capsule, or an injectable. Various dosage forms are envisaged for use in the invention.

The drug products containing the homodimer are preferably in a dosage form suitable for oral use, for example, as tablets, troches, lozenges, aqueous or oily suspensions, dispersible powders or granules, emulsions, hard or soft capsules, or syrups or elixirs. Any appropriate edible substance may be used in the drug products of the invention. Drug products intended for oral use may be prepared according to any method known to the art for the manufacture of drug products and such products may contain one or more agents such as sweetening agents, flavouring agents, colouring agents and preserving agents, e.g. to provide storage-stable and palatable preparations. Tablets contain the homodimer in admixture with pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated to form osmotic therapeutic tablets for controlled release.

Formulations for oral use may also be presented as hard gelatin capsules wherein the homodimer is mixed with an inert solid diluent, for example, calcium carbonate, calcium phosphate or kaolin, or as soft gelatin capsules wherein the homodimer is mixed with water or an oil medium, for example peanut oil, liquid paraffin, or olive oil.

In some embodiments, the homodimer is formulated in an enteric coated capsule for peroral administration (*e.g*. as described in the Examples herein).

Aqueous suspensions contain the homodimer in admixture with excipients suitable for the manufacture of aqueous suspensions. Such excipients are suspending agents, for example sodium carboxymethylcellulose, methylcellulose, hydroxy-propylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more colouring agents, one or more flavouring agents, and one or more sweetening agents, such as sucrose or saccharin.

Oily suspensions may be formulated by suspending the homodimer in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol.

Sweetening agents such as those set forth above, and flavouring agents may be added to provide a palatable oral preparation. The compositions may be preserved by the addition of an antioxidant such as ascorbic acid.

Dispersible powders and granules suitable for preparation of an aqueous suspension by the addition of water provide the homodimer in admixture with a dispersing or wetting agent, suspending agent and one or more preservatives. Suitable dispersing or wetting agents and suspending agents are exemplified by those already mentioned above. Additional excipients, for example sweetening, flavouring and colouring agents, may also be present.

In some embodiments, the homodimer is formulated as a powder for intraoral administration (*e.g.* as described in the Examples herein). Powder formulations suitable for intraoral use include sachets that can be emptied under the tongue.

Formulations suitable for oral administration may be designed to deliver the homodimer in an immediate release manner or in a rate-sustaining manner, wherein the release profile can be delayed, pulsed, controlled, sustained, or delayed and sustained or otherwise modified in such a manner which optimises the therapeutic efficacy of the homodimer.

Formulations suitable for delivery of the homodimer in a rate-sustaining manner are known in the art and include slow release polymers that can be formulated with the homodimer to control their release. Examples of rate-sustaining polymers include degradable and non-degradable polymers that can be used to release the homodimer by diffusion or a combination of diffusion and polymer erosion. Examples of rate-sustaining polymers include hydroxypropyl methylcellulose, hydroxypropyl cellulose, methyl cellulose, ethyl cellulose, and sodium carboxymethyl cellulose.

Formulations suitable for immediate release include fast-dissolving drug delivery systems, such as thin films, fast dissolving tablets (FDTs), and other known dosage forms that can be placed in the mouth, *e.g*. under the tongue. Therefore, in some embodiments, the homodimer is formulated for intraoral administration using a fast-dissolving intraoral drug delivery system.

The homodimer may be formulated in the form of microparticles or nanoparticles, *e.g*. biodegradable polymer particles, wherein the homodimer is adsorbed onto the surface of the particles or entrapped within the polymer matrix of the particles. The properties of these microparticles and nanoparticles and the way in which the homodimer is associated with the particles may be adjusted using methods known to the skilled person in order to tailor the homodimer release properties of the composition.

The pharmaceutical compositions used in the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents.

In some embodiments, the homodimer is formulated or administered with a bulking agent, *e.g.* a carbohydrate bulking agent (such as mannitol, maltose, sucrose, lactose, trehalose, dextran, sorbitol, cellulose, and the like) as an excipient. In some embodiments, the homodimer is formulated or administered with a bulking agent, *e.g*. a carbohydrate bulking agent (such as mannitol, maltose, sucrose, lactose, trehalose, dextran, sorbitol, cellulose, and the like) as the sole excipient. In some embodiments, the homodimer is formulated or administered with mannitol as an excipient or with mannitol as the sole excipient.

### Dosage Regimens

Treatment of a mammalian subject with the homodimer may consist of a single dose or a multiple dose schedule, and each individual dose may consist of a single dosage form or multiple dosage forms. The dosage forms used in the invention may be presented in unit-dose or multi-dose containers.

The homodimer may be administered at any appropriate frequency of dosing. For example, the homodimer may be administered once every day, or more than once every day (for example 2 or 3 times a day). The homodimer may be administered once every week, or more than once every week (for example 2 or 3 times each week). The homodimer may be administered once every month, or more than once every month (for example 2 or 3 times each month).

The homodimer may be administered initially at a loading dose or frequency and then subsequently at a maintenance dose, wherein the loading dose or frequency is different to the maintenance dose or frequency.

The skilled person can identify a suitable dosage regimen via routine clinical investigations. It will be understood that the specific dosage regimen for any particular human subject may be varied and will depend upon a variety of factors including *inter alia* the age, body weight, general health, sex, diet, mode and time of administration, the severity of the particular condition, and the host undergoing therapy.

### Therapeutic and Prophylactic Uses of the Homodimer

The invention relates to treating or preventing a disease or condition in a mammalian subject by administering the homodimer to the mammalian subject. As used herein, the terms "treating" and "preventing" refer to affecting a mammalian subject to obtain a desirable pharmacological and/or physiological effect. The term "treating" refers to ameliorating the effects of an established disease or condition, whether or not diagnosed. The term "preventing" refers to delaying or arresting the development of a disease or condition in a subject that may be predisposed to the disease but who has not yet developed the disease. The terms "administering" and "administration" should be understood to mean providing the homodimer to a mammalian subject, via any appropriate method.

Diseases that are suitable for treatment or prevention with the homodimer include those described and exemplified in WO 2007/017686. The homodimer may be used in the treatment or prevention of diseases or conditions characterised by hyperinsulinaemia, hyperglucagonaemia, glucose intolerance (*e.g*. associated with an abnormal response in an oral glucose tolerance test; OGTT) and/or insulin resistance, or in the treatment or prevention of any diseases or conditions which are caused or exacerbated by hyperinsulinaemia, hyperglucagonaemia, glucose intolerance and/or insulin resistance. The homodimer may be used in the treatment or prevention of hyperinsulinaemia and/or hyperglucagonaemia. The homodimer may be used in the treatment or prevention of diabetes. The homodimer may be used in the treatment or prevention of Type 1 diabetes. The homodimer may be used in the treatment or prevention of Type 2 diabetes. The homodimer may be used in the treatment or prevention of complications associated with diabetes, *e.g*. diabetic retinopathy, diabetic neuropathy, or diabetic nephropathy. These diseases and conditions are listed by way of example and are not exhaustive. Other diseases that are suitable for treatment or prevention with the homodimer include those described in WO 2007/017686.

In some embodiments, the homodimer is used in the treatment or prevention of diabetes (including Type 1 and Type 2 diabetes) in humans via oral administration.

In some embodiments, the homodimer is used in the treatment or prevention of diabetes (including Type 1 and Type 2 diabetes) in humans via peroral administration.

In some embodiments, the homodimer is used in the treatment or prevention of diabetes (including Type 1 and Type 2 diabetes) in humans via intraoral administration, such as buccal or sublingual delivery.

### Monotherapies and combination therapies

As noted elsewhere herein, the homodimer may be used as the sole active ingredient of the drug product administered to the mammalian subject. The homodimer may therefore be formulated or administered as the sole active ingredient of a pharmaceutical composition administered to the mammalian subject. In other words, the homodimer may be formulated in a drug product that does not contain any other active ingredients, in addition to the homodimer.

The homodimer may be formulated or administered in the absence of any other peptide active ingredient, e.g. formulated or administered in a pharmaceutical composition that does not contain any other peptide active ingredient. For example, the homodimer may be formulated or administered in the absence of any peptide comprising or consisting of an amino acid sequence as recited in SEQ ID NO:2 (NIYPSDSYTNYNQKFKD) or SEQ ID NO:3 (CNIYPSDSYTNYNQKFKD), or any multimer thereof. The homodimer may be formulated or administered in the absence of any peptide comprising or consisting of an amino acid sequence as recited in SEQ ID NO:4 (LRGLLPDY) or SEQ ID NO:5 (CLRGLLPDY), or any multimer thereof. The homodimer may be formulated or administered in the absence of any peptide comprising or consisting of an amino acid sequence as recited in any of SEQ ID NOs:2-5, or any multimer thereof. The homodimer may be administered to the mammalian subject without co-administration of a separate drug product comprising any peptide comprising or consisting of an amino acid sequence as recited in any of SEQ ID NOs:2-5, or any multimer thereof.

However, in other embodiments, the homodimer may be formulated or administered in combination with one or more other active ingredients. When the homodimer is administered in combination with another active ingredient, the homodimer and the other active ingredient are preferably both administered in a therapeutically or prophylactically effective amount, as appropriate. The homodimer may be administered either simultaneously with, or before or after, the other active ingredient but sufficiently close together in time so as to provide a combined therapeutic effect. The homodimer may be administered separately, by the same or different route of administration, or together in the same drug product as the other active ingredient(s).

Therefore, the drug products containing the homodimer may further comprise one or more other active ingredients, or the drug products containing the homodimer may be administered alongside one or more further separate drug products containing the one or more other active ingredients. In some embodiments, the homodimer is formulated or administered in combination with one or more other active ingredients, but is not formulated or administered in a drug product that contains any peptide comprising or consisting of an amino acid sequence as recited in SEQ ID NO:2 or SEQ ID NO:3, or any multimer thereof. In some embodiments, the homodimer is formulated or administered in combination with one or more other active ingredients, but is not formulated or administered in combination with any peptide comprising or consisting of an amino acid sequence as recited in SEQ ID NO:4 or SEQ ID NO:5, or any multimer thereof. In some embodiments, the homodimer is formulated or administered in combination with one or more other active ingredients, but is not formulated or administered in combination with any peptide comprising or consisting of an amino acid sequence as recited in any of SEQ ID NOs:2-5, or any multimer thereof.

The homodimer may be formulated or administered in combination with one or more other anti-diabetic agents. Examples of anti-diabetic agents that may be suitable for use in combination with the homodimer include biguanides (e.g., metformin or phenformin), glucosidase inhibitors (e.g,. acarbose or miglitol), insulins (including insulin secretagogues or insulin sensitizers), meglitinides (e.g., repaglinide), sulfonylureas (e.g., glimepiride, glyburide, gliclazide, chlorpropamide and glipizide), biguanide/glyburide combinations (e.g., Glucovance®), thiazolidinediones (e.g., troglitazone, rosiglitazone and pioglitazone), PPAR-alpha agonists, PPAR-gamma agonists, PPAR alpha/gamma dual agonists, glycogen phosphorylase inhibitors, inhibitors of fatty acid binding protein (aP2), DPP-IV inhibitors, SGLT2 inhibitors and GLP-1 receptor modulators. Therefore, in some embodiments, the homodimer is formulated or administered in combination with a second active ingredient selected from biguanides (e.g., metformin or phenformin), glucosidase inhibitors (e.g,. acarbose or miglitol), insulins (including insulin secretagogues or insulin sensitizers), meglitinides (e.g., repaglinide), sulfonylureas (e.g., glimepiride, glyburide, gliclazide, chlorpropamide and glipizide), biguanide/glyburide combinations (e.g., Glucovance®), thiazolidinediones (e.g., troglitazone, rosiglitazone and pioglitazone), PPAR-alpha agonists, PPAR-gamma agonists, PPAR alpha/gamma dual agonists, glycogen phosphorylase inhibitors, inhibitors of fatty acid binding protein (aP2), DPP-IV inhibitors, SGLT2 inhibitors and GLP-1 receptor modulators.

Other active ingredients potentially of use in combination with the homodimer for the treatment or prevention of the diseases and conditions described herein will be known to the skilled person, and the selection of the appropriate agents for use in combination therapy may be made according to conventional pharmaceutical principles. When other active ingredients are employed in combination with the homodimer they may be used for example in amounts as noted in the Physician Desk Reference (PDR) or as otherwise determined by one of ordinary skill in the art.

### General

The term "comprising" encompasses "including" as well as "consisting", *e.g*. a composition "comprising" X may consist exclusively of X or may include something additional, *e.g*. X + Y.

In the present application the standard one-letter code for amino acid residues is used. Where the L or D form is not specified it is to be understood that the amino acid in question has the natural L form.

Various embodiments of the invention are described herein. It will be recognised that the features specified for each embodiment may be combined with other specified features to provide further embodiments.

For the avoidance of doubt, the features of the embodiments of the invention described above will normally apply equally to the different aspects of the invention. For example, it should be understood that the compositions, uses and methods disclosed herein for "the homodimer" (in the sections above describing pharmaceutical compositions, routes of administration, dosages, dosage forms and dosage regimens, therapeutic and prophylactic uses of the homodimer, and monotherapies and combination therapies) are also relevant to the "pharmaceutically acceptable salts" of the homodimer, provided there is no technical reason why such an interpretation is inappropriate, because pharmaceutically acceptable salts of the homodimer may also be used in the compositions, uses and methods of the invention. As a further example, any features described above solely in connection with "compositions" also apply to the uses and methods of the invention, provided there is no technical reason why such an interpretation is inappropriate. As a specific example, if it is stated that "the homodimer may be formulated or administered in the absence of any functional excipient", this equates to a statement that the methods disclosed herein may involve "formulating or administering the homodimer in the absence of any functional excipient", and to a statement that "a pharmaceutically acceptable salt of the homodimer may be formulated or administered in the absence of any functional excipient".

Various aspects and embodiments of the present invention will now be described in more detail by way of example, with particular reference to specific peptides.

### EXAMPLES

The surprising advantageous properties of the disulphide-linked homodimer used in the invention were first suggested by tissue permeation tests performed *in vitro* using an organotypic tissue model derived from human buccal mucosa, and were subsequently corroborated by preliminary clinical testing in human diabetic patients.

### Example 1: Human buccal tissue permeability screening

A human buccal tissue model was used for *in vitro* testing of the permeability of a number of disulphide-linked homodimers.

### Preparation of human buccal tissue model

Human oral mucosa tissue models were prepared in accordance with the standard protocols of a commercial reagent supplier (EpiOral™, MatTek Corporation; for details see http://www.mattek.com/epioral/applications/drug-delivery; see also for example Thakur et al., Drug Development and Industrial Pharmacy, 2007, 33(5), pages 513-52). In brief, the tissue models were prepared in tissue culture wells of 24-well plates containing an inner well (insert) with a microporous membrane base coated with an extracellular matrix preparation, on which the human mucosal cells were seeded. The inner well was submerged in the reagent supplier's recommended serum free culture medium which permeates through the microporous membrane to feed the cells in the insert. The cells were submerged in the culture medium for a few days, after insertion of the inner wells into the tissue culture wells and permeation of the culture medium into the inner wells, in accordance with the reagent supplier's recommendation. The inserts containing the developing tissues were then elevated to the air/liquid interface which induces stratification and differentiation. The resulting human buccal cell cultures were stratified, three dimensional tissues comprising 25-30 cell layers, non-cornified with nucleated cells extending up to the apical surface and with histology similar to that of native buccal tissue.

### Cell permeation experiments

The buccal tissue culture inserts were transferred from the original tissue culture 24-well plates (maintained at 4°C in sealed bags containing 5% CO₂) under sterile conditions into wells of a fresh 24-well plate containing 1.0 ml of the reagent supplier's recommended serum free medium prewarmed to 37°C. The plate was then incubated in a humidified incubator at 37°C in a 5% CO₂ atmosphere for one hour as a pre-equilibration procedure prior to dosing.

After removal of the pre-equilibrated plate from the incubator, any culture medium over the surface of the tissue was carefully removed and replaced with 100µL of appropriately diluted homodimer solution in reagent supplier's recommended serum free culture medium (stock homodimer-containing medium). The plate was then incubated for a further 30 minutes at 37°C in a 5% CO₂ atmosphere. The plate was removed from the incubator and supernatant samples were taken from above the cells, inside the insert, and analysed by HPLC. The main peak areas for each tested peptide in the supernatants were compared to the corresponding peak areas for the tested peptides in the relevant pre-incubation samples of the stock homodimer-containing medium.

Table 1 summarises a preliminary permeability analysis of three disulphide-linked peptide homodimers (Homodimers A-C):

**Table 1. Buccal tissue permeability screening of three peptide solutions**

| | Peak Area by HPLC of Peptides in Solution | | |
|---|---|---|---|
| Homodimer | Pre-overlay | Post-overlay | Transmissibility (%) |
| A | 5398 | 4439 | 17.7% |
| B | 2023 | 2396 | 0% |
| C | 4142 | 243 | 94.1% |

Homodimer A is a disulphide-linked homodimer of monomer peptides consisting of the sequence CNIYPSDSYTNYNQKFKD (SEQ ID NO:3). Homodimer B is a disulphide-linked homodimer of monomer peptides consisting of the sequence CLRGLLPDY (SEQ ID NO:5). Homodimer C is a disulphide-linked homodimer of monomer peptides consisting of the sequence CQQYNSYPLT (SEQ ID NO:1).

The results of these experiments suggest that Homodimer A shows only poor transmissibility (absorption) across the human buccal tissue surface, while Homodimer B did not demonstrate any transmissibility across the human buccal tissue surface under the experimental conditions. The apparent small increase in peak area for Homodimer B post-overlay is believed to be an indicator of degradation products derived from Homodimer B. In contrast, the results suggest that Homodimer C was transmitted efficiently across the human buccal tissue surface.

Further experiments of the same type were conducted with Homodimer C, for which the results are provided in Table 2.

The results of these further experiments confirmed the surprising result for Homodimer C observed in Table 1. Consistent with the preliminary experiments, in these further experiments Homodimer C showed efficient uptake by the human buccal tissue cells in the triplicate cultures.

**Table 2. Buccal tissue permeability of Homodimer C in triplicate cultures**

| | Peak Area by HPLC of Peptides in Solution | | |
|---|---|---|---|
| Culture | Pre-overlay | Post-overlay | Transmissibility (%) |
| Replicate 1 | 4142 | 276 | 93.3% |
| Replicate 2 | 4142 | 539 | 84.5% |
| Replicate 3 | 4142 | Undetectable | 100 % |

The results in Table 1 and Table 2 suggest that it may be possible to successfully administer Homodimer C to human patients via buccal administration, or other transmucosal delivery routes.

### Example 2: Therapeutic efficacy testing in human patients

Homodimer C was tested in human clinical trials to determine therapeutic efficacy of transmucosal delivery. Both intraoral (sublingual) delivery and peroral delivery were tested. Two further groups of patients were administered an injectable preparation of Homodimer C to allow a comparison of the effects of transmucosal delivery to the effects of injection delivery. Surprisingly, significant therapeutic efficacy was observed for both of the mucosal administration routes tested (intraoral and peroral). Furthermore, both of the mucosal administration routes tested showed superior therapeutic efficacy compared to injection delivery.

### Preparation of enteric coated capsules (for delivery via the upper intestine)

*Preparation of peptide in mannitol mixture*: The formulation included only Homodimer C and mannitol as a bulking agent. A 2% peptide to mannitol (w/w) composition was mixed for 1 hour at 120rpm in a horizontally revolving tubular blender with baffles to prevent contents from slipping around on rotation. Uniformity of blend was tested by removing samples from various parts of the blend and testing for peptide content by appropriate HPLC procedures. The capsules were filled using a calibrated filling machine and closed.

*Preparation of enteric coating*: OPADRY 20M29149 which is a polyvinyl alcohol based formulation from Colorcon was used. The proprietary enteric coating powder was mixed in sterile water heated to 35°C by slowly adding 20gm powder to 100gm water in a container using a magnetic stirrer. The powder was slowly added to the water over a period of 30 minutes with continued stirring for 1 hour.

*Enteric coating of filled capsules*: The filled capsules were inserted into platen holders that grip the small diameter end of the capsules. The capsules were dipped in the coating solution to 60% of their length from the small diameter end. They were dried in airstream of 60cm per second at 23 to 28°C for 1 hour. The airstream was measured by an impeller anemometer. The capsules were then inserted by the coated end into a second set of larger diameter platens and dipped in the coating solution to ensure coating overlap at the capsule joint. Coated capsules were dried for an hour as previously described. The capsules were then packaged and stored. The coated capsules could withstand up to pH 5 or 6 and disintegrated quickly at neutral or alkaline pH.

### Preparation of uncoated capsules (for sublingual delivery)

A mixture of Homodimer C with mannitol was prepared as described above, and used to fill uncoated capsules. For sublingual delivery, the tip of the uncoated capsule was cut and the contents of the capsule tipped under the tongue. The powder was absorbed within seconds.

### Results of clinical trials

Diabetic patients treated with oral anti-diabetic drugs (*e.g*. sulfonylurea type drugs) or insulin were recruited and randomised to four patient groups: two injectable peptide groups (Groups 1 and 2) and to two oral peptide dosage groups, for delivery of Homodimer C either by sublingual administration (Group 3) or via enteric coated capsules (Group 4). The four treatment groups are summarised in Table 3.

Each group was administered five doses of Homodimer C. Homodimer C was administered once weekly (at days 0, 7, 14, 21 and 28 of the study) at a dose of 2 mg (which equates to approximately 0.03 mg/kg for a notional 70kg subject) for all four groups. The homodimer injections were intramuscular, generally into the upper arm of the subjects. The patients' current treatment was not withdrawn during the study. HbA1c levels were determined in advance of the first dose and at day 35 of the study.

**Table 3. Clinical trial groups and population characteristics**

| *Group (Number=n)* | *Peptide Format* | *Previous Treatment* | *Age (Mean ± sd.)* | *BMI (Mean ± sd.)* | *Years Diagnosed (Mean ± sd.)* |
|---|---|---|---|---|---|
| 1 (n=17) | Injection | Insulin only | 49.1 ± 8.2 | 24.7 ± 3.8 | 7.3 ± 4.1 |
| 2 (n=18) | Injection | Oral anti-diabetics and/or insulin | 49.8 ± 11.1 | 25.2 ± 3.9 | 5.1 ± 2.3 |
| 3 (n= 7) | Sublingual | Oral anti-diabetics and/or insulin | 49.0 ± 7.8 | 24.1 ± 2.8 | 6.3 ± 5.6 |
| 4 (n=7) | Capsule | Oral anti-diabetics and/or insulin | 46.6 ± 10.7 | 25.8 ± 2.8 | 5.4 ± 2.3 |

The results in Table 4 below indicate that Homodimer C is therapeutically effective in human diabetic subjects, because for each of Groups 1-4 at day 35 the mean HbA1c levels were significantly reduced relative to the mean baseline HbA1c levels for each group.

The results in Table 4 also indicate that Homodimer C is therapeutically effective when administered via the intraoral (sublingual) or peroral routes, because at day 35 the mean HbA1c levels were significantly reduced relative to the mean baseline HbA1c levels for each group.

The results in Table 4 further indicate that Homodimer C is significantly more effective when administered via the intraoral (sublingual) or peroral routes than when administered parenterally (by injection).

**Table 4. Clinical trial results**

| *Group* | *Peptide Format* | *HbA1c % Baseline (Mean ± sd.)* | *HbA1c % Day 35 (Mean ± sd.)* | *HbA1c % Change* | *P Value* |
|---|---|---|---|---|---|
| 1 | Injection | 11.34 ± 2.6 | 10.74 ± 2.0 | -0.60 | P=0.045 |
| 2 | Injection | 10.32 ± 2.24 | 9.57 ± 1.79 | -0.75 | P=0.033 |
| 3 | Sublingual | 9.18 ± 2.64 | 7.85 ± 2.03 | -1.33 | P=0.005 |
| 4 | Capsule | 9.30 ± 1.65 | 8.12 ± 1.04 | -1.18 | P=0.016 |

HbA1c (glycated haemoglobin) levels are the preferred measure for monitoring efficacy of diabetes treatments because they provide an indication of long-term serum glucose regulation. HbA1c levels are proportional to average blood glucose concentrations over the previous four weeks to three months. These results are therefore of direct clinical relevance and indicate that Homodimer C is a promising candidate therapeutic for the treatment or prevention of diabetes and related conditions by transmucosal delivery, and in particular via peroral or intraoral delivery. These results are especially surprising because of the difficulties normally encountered for oral delivery of peptide therapeutics, and in light of the fact that Homodimer C is not chemically modified and was not administered in conjunction with any functional excipients. Furthermore, these results are particularly surprising because the oral delivery of Homodimer C outperformed the parenteral delivery of the homodimer.

As noted elsewhere herein, the surprising results presented herein for oral delivery of the homodimer are believed to be relevant to administration of the homodimer via the nasal, pulmonary, vaginal and rectal mucosa, for example because of similarities in the structures and properties of the different mucosa (*e.g*. similarities in the epithelial cell layers of the different mucosa). Indeed, in some respects oral delivery of peptide therapeutics is considered likely to be more difficult than delivery via the nasal, pulmonary, vaginal or rectal mucosa (for example, due to differences in the surface areas and barrier properties of the different mucosa; *e.g*. see Table 3.4 in Delivery Technologies for Biopharmaceuticals: Peptides, Proteins, Nucleic Acids and Vaccines, Jorgensen & Nielsen, 2009).

## Claims

1. A homodimer consisting of covalently linked monomer peptides, each monomer consisting of the amino acid sequence CQQYNSYPLT (SEQ ID NO:1), wherein the monomer peptides are linked by a disulphide bond between the N-terminal cysteine residues of the monomers, or a pharmaceutically acceptable salt of the homodimer, for use in the treatment or prevention of hyperinsulinaemia, hyperglucagonaemia, glucose intolerance, and/or insulin resistance in a mammalian subject by oral administration.

2. The homodimer for use of claim 1, for use in the treatment or prevention of diabetes.

3. The homodimer for use of claim 2, for use in the treatment or prevention of Type 1 diabetes.

4. The homodimer for use of claim 2, for use in the treatment or prevention of Type 2 diabetes.

5. The homodimer for use of any preceding claim, wherein the oral administration is peroral or intraoral.

6. The homodimer for use of claim 5, wherein the intraoral administration is sublingual administration or buccal administration.

7. The homodimer for use of any preceding claim, wherein the subject is a human subject.

8. The homodimer for use of any preceding claim, wherein the homodimer is administered in a drug product comprising the homodimer as the sole active ingredient.

9. The homodimer for use of any of claims 1-7, wherein the homodimer is administered in combination with one or more other active ingredients.

10. The homodimer for use of any preceding claim, wherein the homodimer is administered in combination with one or more other anti-diabetic agents.

11. The homodimer for use of any preceding claim, wherein the homodimer is administered in a pharmaceutical composition containing one or more pharmaceutically acceptable excipients.

12. The homodimer for use of claim 11, wherein the homodimer is administered in a pharmaceutical composition containing one or more non-functional excipients, or wherein the homodimer is administered in a pharmaceutical composition containing only non-functional excipients.

13. The homodimer for use of claim 11, wherein the homodimer is administered in a pharmaceutical composition containing one or more functional excipients, or wherein the homodimer is co-administered to the mammalian subject with separate functional excipients.

14. The homodimer for use of claim 11 or claim 12, wherein the homodimer is administered to the mammalian subject without co-administration of separate functional excipients.

15. The homodimer for use of any of claims 1-14, wherein either:
(a) the homodimer peptide is administered to the mammalian subject at from 0.1 to 10 mg per dose; or
(b) the homodimer peptide is administered to the mammalian subject at a dose of from 0.001 to 2 mg/kg.

16. A pharmaceutical composition comprising: (i) a homodimer consisting of covalently linked monomer peptides, each monomer consisting of the amino acid sequence CQQYNSYPLT (SEQ ID NO:1), wherein the monomer peptides are linked by a disulphide bond between the N-terminal cysteine residues of the monomers, or a pharmaceutically acceptable salt of the homodimer; and (ii) one or more pharmaceutically acceptable excipients, for use in the treatment or prevention of hyperinsulinaemia, hyperglucagonaemia, glucose intolerance, and/or insulin resistance in a mammalian subject by oral administration according to any of claims 1-15.

## Patentansprüche

1. Homodimer, bestehend aus kovalent verknüpften Monomerpeptiden, wobei jedes Monomer aus der Aminosäuresequenz CQQYNSYPLT (SEQ ID NO:1) besteht, wobei die Monomerpeptide über eine Disulfidbindung zwischen den N-terminalen Cysteinresten der Monomere verknüpft sind, oder ein pharmazeutisch unbedenkliches Salz des Homodimers, zur Verwendung bei der Behandlung oder Prävention von Hyperinsulinämie, Hyperglucagonämie, Glucoseintoleranz und/oder Insulinresistenz bei einem Säugetierpatienten durch orale Verabreichung.

2. Homodimer zur Verwendung nach Anspruch 1 zur Verwendung bei der Behandlung oder Prävention von Diabetes.

3. Homodimer zur Verwendung nach Anspruch 2 zur Verwendung bei der Behandlung oder Prävention von Typ-1-Diabetes.

4. Homodimer zur Verwendung nach Anspruch 2 zur Verwendung bei der Behandlung oder Prävention von Typ-2-Diabetes.

5. Homodimer zur Verwendung nach einem der vorhergehenden Ansprüche, wobei die orale Verabreichung peroral oder intraoral erfolgt.

6. Homodimer zur Verwendung nach Anspruch 5, wobei es sich bei der intraoralen Verabreichung um sublinguale Verabreichung oder bukkale Verabreichung handelt.

7. Homodimer zur Verwendung nach einem der vorhergehenden Ansprüche, wobei es sich bei dem Patienten um einen menschlichen Patienten handelt.

8. Homodimer zur Verwendung nach einem der vorhergehenden Ansprüche, wobei das Homodimer in einem Arzneistoffprodukt, das das Homodimer als einzigen Wirkstoff umfasst, verabreicht wird.

9. Homodimer zur Verwendung nach einem der Ansprüche 1-7, wobei das Homodimer in Kombination mit einem oder mehreren anderen Wirkstoffen verabreicht wird.

10. Homodimer zur Verwendung nach einem der vorhergehenden Ansprüche, wobei das Homodimer in Kombination mit einem oder mehreren Antidiabetika verabreicht wird.

11. Homodimer zur Verwendung nach einem der vorhergehenden Ansprüche, wobei das Homodimer in einer pharmazeutischen Zusammensetzung, die einen oder mehrere pharmazeutisch unbedenkliche Hilfsstoffe enthält, verabreicht wird.

12. Homodimer zur Verwendung nach Anspruch 11, wobei das Homodimer in einer pharmazeutischen Zusammensetzung, die einen oder mehrere nichtfunktionelle Hilfsstoffe enthält, verabreicht wird oder wobei das Homodimer in einer pharmazeutischen Zusammensetzung, die nur nichtfunktionelle Hilfsstoffe enthält, verabreicht wird.

13. Homodimer zur Verwendung nach Anspruch 11, wobei das Homodimer in einer pharmazeutischen Zusammensetzung, die einen oder mehrere funktionelle Hilfsstoffe enthält, verabreicht wird oder wobei das Homodimer dem Säugetierpatienten zusammen mit separaten funktionellen Hilfsstoffen verabreicht wird.

14. Homodimer zur Verwendung nach Anspruch 11 oder 12, wobei das Homodimer dem Säugetierpatienten ohne gleichzeitige Verabreichung separater funktioneller Hilfsstoffe verabreicht wird.

15. Homodimer zur Verwendung nach einem der Ansprüche 1-14, wobei entweder:
(a) das Homodimerpeptid dem Säugetierpatienten in einer Menge von 0,1 bis 10 mg pro Dosis verabreicht wird oder
(b) das Homodimerpeptid dem Säugetierpatienten in einer Menge von 0,001 bis 2 mg/kg verabreicht wird.

16. Pharmazeutische Zusammensetzung, umfassend: (i) ein Homodimer, bestehend aus kovalent verknüpften Monomerpeptiden, wobei jedes Monomer aus der Aminosäuresequenz CQQYNSYPLT (SEQ ID NO:1) besteht, wobei die Monomerpeptide über eine Disulfidbindung zwischen den N-terminalen Cysteinresten der Monomere verknüpft sind, oder ein pharmazeutisch unbedenkliches Salz des Homodimers; und (ii) einen oder mehrere pharmazeutisch unbedenkliche Hilfsstoffe, zur Verwendung bei der Behandlung oder Prävention von Hyperinsulinämie, Hyperglucagonämie, Glucoseintoleranz und/oder Insulinresistenz bei einem Säugetierpatienten durch orale Verabreichung nach einem der Ansprüche 1-15.

## Revendications

1. Homodimère constitué de peptides monomères liés de façon covalente, chaque monomère étant constitué de la séquence d'acides aminés CQQYNSYPLT (SEQ ID NO : 1), dans lequel les peptides monomères sont liés par une liaison disulfure entre les résidus cystéine N-terminaux des monomères, ou sel pharmaceutiquement acceptable de l'homodimère, pour utilisation dans le traitement ou la prévention de l'hyperinsulinémie, l'hyperglucagonémie, l'intolérance au glucose et/ou l'insulinorésistance chez un sujet mammifère par administration orale.

2. Homodimère pour utilisation selon la revendication 1, pour utilisation dans le traitement ou la prévention du diabète.

3. Homodimère pour utilisation selon la revendication 2, pour utilisation dans le traitement ou la prévention du diabète de type 1.

4. Homodimère pour utilisation selon la revendication 2, pour utilisation dans le traitement ou la prévention du diabète de type 2.

5. Homodimère pour utilisation selon l'une quelconque des revendications précédentes, où l'administration orale est perorale ou intraorale.

6. Homodimère pour utilisation selon la revendication 5, où l'administration intraorale est l'administration sublinguale ou l'administration buccale.

7. Homodimère pour utilisation selon l'une quelconque des revendications précédentes, où le sujet est un sujet humain.

8. Homodimère pour utilisation selon l'une quelconque des revendications précédentes, où l'homodimère est administré dans un produit pharmaceutique comprenant l'homodimère en tant que seule substance active.

9. Homodimère pour utilisation selon l'une quelconque des revendications 1 à 7, où l'homodimère est administré en combinaison avec une ou plusieurs autres substances actives.

10. Homodimère pour utilisation selon l'une quelconque des revendications précédentes, où l'homodimère est administré en combinaison avec un ou plusieurs autres agents antidiabétiques.

11. Homodimère pour utilisation selon l'une quelconque des revendications précédentes, où l'homodimère est administré dans une composition pharmaceutique contenant un ou plusieurs excipients pharmaceutiquement acceptables.

12. Homodimère pour utilisation selon la revendication 11, où l'homodimère est administré dans une composition pharmaceutique contenant un ou plusieurs excipients non fonctionnels, ou l'homodimère est administré dans une composition pharmaceutique contenant uniquement des excipients non fonctionnels.

13. Homodimère pour utilisation selon la revendication 11, où l'homodimère est administré dans une composition pharmaceutique contenant un ou plusieurs excipients fonctionnels, ou l'homodimère est coadministré au sujet mammifère avec des excipients fonctionnels séparés.

14. Homodimère pour utilisation selon la revendication 11 ou la revendication 12, où l'homodimère est administré au sujet mammifère sans coadministration d'excipients fonctionnels séparés.

15. Homodimère pour utilisation selon l'une quelconque des revendications 1 à 14, où soit :
(a) le peptide homodimère est administré au sujet mammifère à 0,1 à 10 mg par dose ; ou
(b) le peptide homodimère est administré au sujet mammifère à une dose de 0,001 à 2 mg/kg.

16. Composition pharmaceutique comprenant : (i) un homodimère constitué de peptides monomères liés de façon covalente, chaque monomère étant constitué de la séquence d'acides aminés CQQYNSYPLT (SEQ ID NO : 1), dans laquelle les peptides monomères sont liés par une liaison disulfure entre les résidus cystéine N-terminaux des monomères, ou un sel pharmaceutiquement acceptable de l'homodimère ; et (ii) un ou plusieurs excipients pharmaceutiquement acceptables, pour utilisation dans le traitement ou la prévention de l'hyperinsulinémie, l'hyperglucagonémie, l'intolérance au glucose et/ou l'insulinorésistance chez un sujet mammifère par administration orale selon l'une quelconque des revendications 1 à 15.
